# EUROPÄISCHE PATENTSCHRIFT

(11) **EP 3 554 578 B1**
(45) Veröffentlichungstag und Bekanntmachung des Hinweises auf die Patenterteilung: **07.08.2024**
(21) Anmeldenummer: 17828684.5
(22) Anmeldetag: 14.12.2017
(51) Int. Cl.: A61M 1/16, A61M 1/36, A61M 1/34

(54) **BLUTFÜHRUNGSVORRICHTUNG ZUR DURCHFÜHRUNG EINER EXTRAKORPORALEN BLUTBEHANDLUNG, BLUTBEHANDLUNGSSYSTEM**
BLOOD-GUIDING DEVICE FOR CARRYING OUT AN EXTRACORPOREAL BLOOD TREATMENT, BLOOD TREATMENT SYSTEM
DISPOSITIF DE CONDUITE DE SANG DESTINÉ À EFFECTUER UN TRAITEMENT DE SANG EXTRACORPOREL, SYSTÈME DE TRAITEMENT DE SANG

(30) Priorität: 15.12.2016 DE 102016014892
(43) Veröffentlichungstag der Anmeldung: 23.10.2019
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Ricker, Mathias
(86) Internationale Anmeldenummer: PCT/EP2017/082783
(87) Internationale Veröffentlichungsnummer: WO 2018/109071

(56) Entgegenhaltungen:
- WO-A1-2016/103290
- US-A1- 2005 182 349
- US-A1- 2005 236 329
- US-A1- 2009 099 498
- US-A1- 2010 288 703

## Beschreibung

Die vorliegende Erfindung betrifft eine Blutführungsvorrichtung zum Zusammenwirken mit einer Blutbehandlungsvorrichtung sowie ein Blutbehandlungssystem.

Auf dem Gebiet der extrakorporalen Blutbehandlung ist es bekannt, verschiedene Therapien in einer einzigen Therapie zusammenzuführen, welche beispielsweise mittels einer einzigen medizinischen Behandlungsvorrichtung beziehungsweise einem einzigen medizinischen Behandlungssystem durchgeführt werden kann. Mit anderen Worten wird dabei mit mindestens zwei separaten Blutbehandlungselementen in einem gemeinsamen extrakorporalen Blutkreislauf auf das entnommene Blut eingewirkt, vorzugsweise auf unterschiedliche Weise. Eine solche Therapie wird als Kombinationstherapie bezeichnet. Üblicherweise werden Therapien kombiniert, deren medizinische Indikationen in ursächlichem Zusammenhang stehen und die deshalb häufig gemeinsam auftreten. Darüber hinaus bietet sich die Kombination von Therapien unterschiedlicher Indikationen auch an, wenn die Behandlungstechnik eine solche Zusammenführung durch vorteilhafte Synergien rechtfertigt.

So gibt es beispielsweise im Gebiet der extrakorporalen Blutbehandlungen die Kombination von Nierenersatztherapien mit anderen extrakorporalen Bluttherapien. Beispielsweise werden Behandlungen der Akutdialyse (CRRT) wie etwa Hämodialyse (HD), Hämodifiltration (HDF), Hämofiltration (HF), Hämoperfusion (HP) oder ISO-UF in einem gemeinsamen extrakorporalen Blutkreislauf in Kombination mit Behandlungen der extrakorporalen Membranoxygenierung, bzw. -CO2-Entfernung angewendet. Üblicherweise werden ein Dialysator und ein Blutbehandlungselement für eine weitere extrakorporale Bluttherapie, wie zum Beispiel ein Gasaustauscher, dazu in einem gemeinsamen extrakorporalen Blutkreislauf in Serie angeordnet.

Ferner ist es bekannt, dass mit steigenden Flussraten die Wirksamkeit des Gasaustauschs, insbesondere für die Entfernung von CO2 aus dem Blut oder für die Anreicherung von 02 im Blut, steigt.

Der Erfinder hat erkannt, dass die zwangsweise Kopplung der Flussraten im Dialysator sowie im Gasaustauscher durch die serielle Anordnung der beiden Komponenten unabhängig von ihrer Reihenfolge für den Betrieb der Behandlungsvorrichtung mit einem wirksamen Gasaustausch problematisch ist. Beispielsweise gehen die in den kontinuierlichen Behandlungsverfahren der Akutdialyse (CRRT) verwendeten Flussraten üblicherweise nicht über 200-300 ml/min hinaus. Dagegen ist die Effizienz der genannten Lungenunterstützungstherapien stark vom Blutfluss abhängig. Im Falle der extrakorporalen Membranoxygenierung (ECMO) kann die Oxygenierungsrate unterhalb von 500 ml/min Blutflussrate nahe Null liegen. Üblicherweise liegen die verwendeten Blutflussraten bei reinen ECMO-Verfahren deshalb deutlich über 1 l/min. CO2-Entfernung ist bereits bei niedrigeren Blutflussraten möglich, jedoch beginnt auch hier der üblicherweise genutzte Bereich erst ab etwa 500 ml/min. Eine Kombination dieser Nierenersatz- und Lungenunterstützungstherapien ist somit im Stand der Technik stets damit verbunden, dass eine der zugrunde liegenden Indikationen mit verminderter Effizienz behandelt wird.

Das genannte Problem beschränkt sich jedoch nicht auf die Kombination von Nierenersatz- und Lungenunterstützungstherapien, sondern tritt stets dann auf, wenn für eine extrakorporale Bluttherapie in einem extrakorporalen Blutkreislauf ein Dialysator in Kombination mit einem Blutbehandlungselement für eine weitere Therapie betrieben wird, wobei der Dialysator und das Blutbehandlungselement für eine jeweils optimale Behandlungseffizienz unterschiedliche Anforderungen an den Blutfluss stellen.

Aus US 2005/182349 ist ein Blutbehandlungssystem mit einem Dialysator, einem Oxygenator und einem Bioreaktor bekannt. Ein Rezirkulationskreislauf ist vorgesehen, der den Oxygenator und den Bioreaktor umfasst. WO 2016/103290 offenbart ein Blutbehandlungssystem mit einem Hämofilter und einem Oxygenator, wobei eine Nebenleitung vorgesehen ist, die den Hämofilter umgeht.

Die vorliegende Erfindung hat zur Aufgabe, die zuvor genannten Nachteile zu überkommen und eine extrakorporale Blutbehandlung unter Verwendung einer Kombination von einem Dialysator und einem weiteren Blutbehandlungselement (Kombinationstherapie) im optimalen Effizienzbereich jeder Teiltherapie zu ermöglichen.

Erfindungsgemäß wird die Aufgabe durch die Vorrichtung und das System gemäß der unabhängigen Ansprüche gelöst. Die Unteransprüche enthalten zudem jeweils vorteilhafte Ausführungen der Erfindung.

Die erfindungsgemäße Blutführungsvorrichtung zum Zusammenwirken mit einer erfindungsgemäßen Blutbehandlungsvorrichtung weist eine Hauptblutleitung zur fluidischen Konnektion mit einem Dialysator sowie zur fluidischen Konnektion mit einem Blutbehandlungselement stromab des Dialysators, wobei die Hauptblutleitung an einem Ende einen Blutentnahmeport zur Konnektion mit einem Blutentnahmezugang eines Patienten und an einem anderen Ende einen Blutrückgabeport zur Konnektion mit einem Blutrückgabezugang des Patienten aufweist; mindestens eine Nebenleitung, welche an einer ersten Verzweigung von der Hauptblutleitung abführt und sich an einer zweiten Verzweigung wieder mit der Hauptblutleitung vereinigt; und einen oder mehrere Pumpanordnungsabschnitte, ausgebildet zum Einwirken der Pumpanordnung der Blutbehandlungsvorrichtung, auf.

Erfindungsgemäß wird Blut in zwei Nebenleitungen geführt, wobei die erste Nebenleitung an einer ersten Verzweigung stromauf des Dialysators von der Hauptblutleitung abgeführt wird und sich an einer zweiten Verzweigung stromab des Dialysators sowie stromauf des Behandlungselements wieder mit der Hauptblutleitung vereinigt. Die zweite Nebenleitung ist an einer Rezirkulationsverzweigung stromab des Blutbehandlungselements von der Hauptblutleitung abgeführt und mündet in einem Rezirkulationsrückgabeport. Der Rezirkulationsrückgabeport ist hierbei in der ersten Nebenleitung stromauf der zweiten Verzweigung angeordnet.

Gemäß einer nicht beanspruchten alternativen Ausführungsform kann der Rezirkulationsrückgabeport auch in der Hauptblutleitung stromauf der Konnektionsstelle für das Blutbehandlungselement sowie stromab der Konnektionsstelle für den Dialysator angeordnet sein.

Bei diesen Ausführungsformen kann die zweite Nebenleitung das Blut durch das Blutbehandlungselement für die weitere Blutbehanldungstherapie rezirkuklieren und somit zur Erhöhung der Effizienz dieser Teiltherapie beitragen.

Das erfindungsgemäße Blutbehandlungssystem weist eine erfindungsgemäße Blutführungsvorrichtung und eine Blutbehandlungsvorrichtung auf. Dabei weist die Blutbehandlungsvorrichtung eine Steuerungsvorrichtung und eine Pumpanordnung, welche zur Erzeugung von Blutflüssen in der Hauptblutleitung sowie in der mindestens einen Nebenleitung eingerichtet ist, auf, wobei die Pumpanordnung wenigstens zwei Elemente umfasst, die auf den Fluss in der Hauptleitung und in der mindestens einen Nebenleitung der Blutführungsvorrichtung wirken, und wobei die Steuerungsvorrichtung konfiguriert ist, die Pumpanordnung derart zu betreiben, dass eine erste Blutflussrate im Dialysator (Dialysatorflussrate) von einer zweiten Blutflussrate in dem Blutbehandlungselement entkoppelt ist. Darüber hinaus ist die Steuerungsvorrichtung dazu konfiguriert, die Pumpanordnung derart zu betreiben, dass die Blutflussrate in mindestens einem Abschnitt der Hauptblutleitung zumindest von einer der Blutflussraten in den Nebenleitungen unabhängig ist.

Mit anderen Worten erlauben die erfindungsgemäßen Blutbehandlungs- und Blutführungsvorrichtungen sowie das erfindungsgemäße Blutbehandlungssystem den Betrieb einer extrakorporalen Blutbehandlung unter Benutzung eines gemeinsamen extrakorporalen Blutkreislaufs bei Serienschaltung eines Dialysators mit einem stromab des Dialysators angeordneten Behandlungselement für eine weitere extrakorporale Bluttherapie mit Hinblick auf die Effizienz der Behandlung zu verbessern. Dabei ist für die weitere extrakorporale Bluttherapie ein höherer Blutfluss sinnvoll, als für die Therapie im Dialysator.

Das Blutbehandlungselement für die weitere extrakorporale Therapie kann ein Gasaustauscher zur O2-Anreicherung und/oder CO2-Entfernung sein. Das Blutbehandlungselement kann auch eine Adsorberkartusche zur therapeutischen Apherese sein. Das Blutbehandlungselement kann auch ein diagnostisches Element sein, welches Parameter des Bluts bestimmen kann, zur Feststellung der Existenz von pathologischen Veränderungen des Bluts. Alle, auf das Blut wirkenden Behandlungselemente können das Blutbehandlungselement im Sinne dieser Beschreibung darstellen. Die Wirkung kann dabei eine mechanische, eine chemische, eine physikalische oder sonstige Wirkung sein.

Im Sinne dieser Beschreibung kann "Therapie" nicht nur Heilung umfassen, sondern zumindest auch Linderung, Symptomtherapie, Verzögerung, Entwöhnung und Diagnose. Insbesondere kann unter Bluttherapie jegliche Wirkung auf das Blut oder Veränderung des Blutes verstanden werden, wie etwa das Hinzufügen von Substanzen in das Blut oder das Entnehmen von Substanzen aus dem Blut, welche eine der oben genannten oder eine entsprechende Wirkung hervorrufen kann.

Die Benutzung eines gemeinsamen extrakorporalen Blutkreislaufs für beide Behandlungen im Sinne einer Kombinationstherapie ist wünschenswert, da hiermit die invasiven Schritte der Blutentnahme sowie der Blutrückführung für beide Therapien nur einmal nötig sind und somit der Patient auch nur einmal den damit einhergehenden Behandlungsrisiken ausgesetzt wird.

Bei der Anordnung des Dialysators und des Blutbehandlungselements in Serie in einem extrakorporalen Blutkreislauf sind grundsätzlich beide Reihenfolgen möglich. Durchströmt das Blut jedoch beispielsweise im Falle der CO2-Entfernung als weitere extrakorporale Blutbehandlung zuerst den Gasaustauscher und danach den Dialysator, kann das nach Durchlaufen des Gasaustauschers bereits CO2-arme Blut im Dialysator eine Wiederanreicherung mit CO2 erfahren. Dies wird bewirkt durch den Konzentrationsgradienten über die Dialysatormembran, da Dialyselösungen üblicherweise Bicarbonat enthalten, in welchem CO2 gepuffert vorliegt. Wird der Gasaustauscher vom Blut erst nach dem Dialysator passiert, kommt es zu dieser Wiederanreicherung nicht.

Die Blutbehandlungsvorrichtung kann die wiederverwendbare Maschinenseite des Blutbehandlungssystems darstellen. Die Blutführungsvorrichtung kann ein Blutschlauchset oder eine Kassette mit Blutleitungen oder eine Kombination aus Blutschläuchen und wenigstens einer Kassette mit Blutleitungen zur Aufrüstung auf die Blutbehandlungsvorrichtung darstellen. Die Blutführungsvorrichtung kann hierbei aus hygienischen Gründen als medizinischer Wegwerfartikel ausgebildet sein, welcher nach jeder Behandlung verworfen wird. Insbesondere kann die Blutführungsvorrichtung neben der Blutführung auch einen oder mehrere weitere Fluidführungen aufweisen, wie etwa einen Dialysatkreislauf oder Leitungen zur Führung eines Sweepgases bei Betrieb eines Gasaustauschers.

Die Hauptblutleitung der Blutführungsvorrichtung kann zur Konnektion mit dem Dialysator und/oder zur Konnektion mit dem Blutbehandlungselement jeweils geeignete Konnektoren oder Anschlüsse aufweisen. Die Ausgestaltung dieser Konnektoren, insbesondere zum Anschluss an den Dialysator, kann beispielsweise eine zylindrische Form mit einem Außendurchmesser im Bereich von 10,5 - 12,8 mm aufweisen, sowie einen kegelförmigen Fluidkanal mit einem Innendurchmesser von 6,33 mm am distalen Ende des Konnektors umfassen. Erfindungsgemäß sind aber auch weitere Ausführungen denkbar, die gemäß der Kenntnis des Fachmanns die Anforderungen an die vorgesehenen Flüsse erfüllen. Weiterhin kann die Blutführungsvorrichtung auch den Dialysator und/oder das Blutbehandlungselement umfassen, wenn diese fest mit der Hauptblutleitung verbunden, zum Beispiel verklebt oder verschweißt sind.

Die erfindungsgemäßen Vorrichtungen zur Blutbehandlung und zur Blutführung können dafür vorgesehen sein, zusammenzuwirken und können gemeinsam ein erfindungsgemäßes System zur Blutbehandlung bilden. Das Blutbehandlungssystem kann über die Blutbehandlungsvorrichtung und die Blutführungsvorrichtung hinaus noch weitere Komponenten aufweisen.

Die Vorrichtungen zur Blutbehandlung und zur Blutführung können jeweils zur Zusammenwirkung bestimmte, komplementäre Komponenten aufweisen. So weist die Blutbehandlungsvorrichtung eine Pumpanordnung auf, während die Blutführungsvorrichtung eine oder mehrere Pumpanordnungsabschnitte, ausgebildet zum Einwirken der Pumpanordnung der Blutbehandlungsvorrichtung aufweist.

Optional kann die Blutbehandlungsvorrichtung in einigen Ausführungsformen einen oder mehrere Drucksensoren aufweisen, während die Blutführungsvorrichtung optional in einigen Ausführungsformen einen oder mehrere Druckmessabschnitte aufweisen kann, die zum Abgreifen des Drucks mittels der genannten Drucksensoren der Blutbehandlungsvorrichtung zur Messung des Drucks ausgestaltet sein können. Bei dem Druckmessabschnitt kann es sich um eine flexible Membran handeln oder um eine Ableitung, die durch eine komprimierbare Gassäule den Druck in der Blutführungsvorrichtung auf den Drucksensor übertragen kann.

Ferner kann die Blutbehandlungsvorrichtung optional in einigen Ausführungsformen eine Infusionspumpe zur Zuleitung medizinischer Flüssigkeit oder zwei Infusionspumpen zur Zuleitung medizinischer Flüssigkeit oder drei Infusionspumpen zur Zuleitung medizinischer Flüssigkeit oder vier oder mehr Infusionspumpen zur Zuleitung medizinischer Flüssigkeit aufweisen, während die Hauptblutleitung der Blutführungsvorrichtung optional in einigen Ausführungsformen einen oder mehrere Zugabeports für medizinische Flüssigkeit zur Antikoagulation sowie weiterhin optional einen oder mehrere Zugabeports für Dilutionsflüssigkeit aufweisen kann.

Unter Zugabeport kann hierbei sowohl ein reiner Anschluss oder Konnektor an der Hauptblutleitung der Blutführungsvorrichtung verstanden werden, zum Beispiel als Luer-Lock ausgestaltet, aber auch eine lösbar oder fest verbundene Zugangsleitung zur Hauptblutleitung. Die oben genannten Infusionspumpen der Blutbehandlungsvorrichtung können dazu vorgesehen sein, pumpend auf die mit den Zugabeports verbundenen Zugangsleitungen einzuwirken. Die Zugangsleitungen können dabei jeweils an Flüssigkeitsreservoirs mit der zuzugebenden Flüssigkeit angeschlossen sein, zur Förderung dieser in die Hauptblutleitung mittels der Infusionspumpen.

In einer Ausführungsform schlägt der Erfinder vor, die Hauptblutleitung des gemeinsamen extrakorporalen Blutkreislaufs an einer ersten Verzweigung stromauf des Dialysators zu verzweigen, eine Nebenleitung um den Dialysator herumzuführen und diese erst stromab des Dialysators sowie stromauf des Gasaustauschers an einer zweiten Verzweigung wieder mit der Hauptblutleitung zu vereinen. Hierbei ordnet der Erfinder den Dialysator in der Hauptblutleitung stromauf vom Gasaustauscher an. Dies kann bewirken, dass die zuvor beschriebene CO2-Wiederanreicherung vermieden werden kann.

Weiterhin kann am extrakorporalen Blutkreislauf eine Pumpanordnung angeordnet sein, welche eingerichtet ist zur Erzeugung von Blutflüssen in der Hauptblutleitung sowie in der Nebenleitung. Dazu kann die Pumpanordnung mit einer Steuerungsvorrichtung verbunden sein. Die Steuerungsvorrichtung ist konfiguriert durch entsprechende Signale den Betrieb der Pumpanordnung zu steuern. Im Sinne der gesamten Beschreibung umfasst der Begriff "Steuerung" auch als Alternative die Möglichkeit einer Regelung.

Die Steuerungsvorrichtung ist konfiguriert, um die Pumpanordnung derart zu betreiben, dass eine erste Blutflussrate im Dialysator von einer zweiten Blutflussrate in dem Blutbehandlungselement entkoppelt ist. Unter "entkoppelt" ist hierbei zu verstehen, dass mittels der Steuerung beliebige Flussraten im Dialysator und in dem Blutbehandlungselement erzeugt werden können, ohne dass die Festlegung einer Flussrate die Wahl der anderen Flussrate limitiert.

In weiteren Ausführungsformen kann die Pumpanordnung ausgebildet sein, um in der Hauptblutleitung und in der Nebenleitung voneinander unabhängige Blutflussraten zu erzeugen. Unter "unabhängig" ist hierbei zu verstehen, dass die Festlegung einer der beiden Flussraten keine Auswirkungen auf die Einstellungen der Pumpanordnung zur der Wahl der anderen Flussrate hat.

Der Fachmann erkennt, dass die Pumpanordnung auf vielfältige Weise ausgeführt sein kann, um auf die oben genannte Weise zu wirken.

Im Allgemeinen weist die Pumpanordnung wenigstens zwei Elemente auf, die auf den Fluss in den beiden Leitungsabschnitten wirken. Wenigstens eines dieser Elemente ist dabei üblicherweise ein aktives Element durch das der Fluss in einem Leitungselement hervorrufbar ist, beispielsweise eine Pumpe. Das zweite der wenigstens zwei Elemente kann ebenfalls ein aktives Element zur Erzeugung eines Flusses sein oder ein passives Element, dessen Wirkung darin bestehen kann, dass der Fluss durch das Element festgelegt oder einstellbar ist. Bei diesem zweiten Element kann es sich beispielsweise um eine Drossel oder ein Ventil handeln.

Beispielsweise kann die Pumpanordnung aus einer okkludierenden Blutpumpe in der Hauptblutleitung stromauf der ersten Verzweigung und einer weiteren okkludierenden Blutpumpe stromab der ersten Verzweigung und stromauf des Dialysators bestehen. Weitere Ausführungsbeispiele der Pumpanordnung werden in den Figuren und in der Beschreibung der Figuren aufgeführt. Die Erfindung umfasst jedoch über die beispielhaft beschriebenen Ausführungsformen hinaus auch alle anderen Pumpanordnungen, welche in der Lage sind, Blut durch die Hauptblutleitung und/oder die mindestens eine Nebenleitung zu führen.

In weiteren Ausführungsformen der Erfindung kann die Pumpanordnung ferner dazu eingerichtet sein, einen Blutfluss in einer zweiten Nebenleitung zu erzeugen. Die Steuerungsvorrichtung kann in diesem Fall konfiguriert sein, die Pumpanordnung derart zu betreiben, dass die Blutflussrate in mindestens einem Abschnitt der Hauptblutleitung zumindest von einer der Blutflussraten in den Nebenleitungen unabhängig ist.

In einer weiteren Ausführungsform verzweigt sich die Hauptblutleitung des gemeinsamen extrakorporalen Blutkreislaufs an einer ersten Verzweigung stromab des Blutbehandlungselements für die weitere extrakorporale Blutbehandlung, eine Nebenleitung führt um das Blutbehandlungselement herum und diese vereint sich stromauf des Blutbehandlungselements sowie stromab des Dialysators wieder mit der Hauptblutleitung. In diesem Fall erwirkt die Pumpanordnung mit dem Blutfluss in der Nebenleitung eine Rezirkulation des Blutflusses über das Blutbehandlungselement. Die Blutflussrate im Blutbehandlungselement erhöht sich im Vergleich zur Blutflussrate im Dialysator um den Betrag des Blutflusses in der Nebenleitung, was zu der erfindungsgemäßen Entkopplung der beiden Flüsse und damit zur Lösung der Aufgabe führt.

Im Falle der CO2-Entfernung wird das extrakorporale Blut beim Durchlaufen des Gasaustauschers zunächst hauptsächlich um das frei im Plasma verfügbare CO2 vermindert. Anschließend wird aus dem natürlichen CO2-Puffersystem des Bluts erneut freies CO2 ins Plasma abgegeben. Die anfängliche Verminderung des Partialdrucks des freien CO2 wird somit nach einiger Zeit wieder ausgeglichen. Der Erfinder hat erkannt, dass das bereits im Gasaustauscher behandelte Blut somit nach kurzer Zeit einer erneuten Behandlung zugänglich ist und sich aus diesem Grund die oben beschriebene Rezirkulation durch den Gasaustauscher lohnen kann.

Bei extrakorporalen Bluttherapien können Maßnahmen ergriffen werden, die einer Koagelbildung des Blutes entgegengewirken. Regelmäßig wird der Patient hierzu systemisch mit einer antikoagulierend wirkenden Substanz, beispielsweise Heparin behandelt, oder es erfolgt eine lokale Antikoagulation im extrakorporalen Blutkreislauf, beispielsweise mittels Heparin oder Citrat- und Calciumzugabe (CiCa-Antikoagulation). Verbreitet sind auch antikoagulierende Beschichtungen der blutführenden Komponenten des extrakorporalen Blutkreislaufes. Das Verfahren mittels CiCa-Antikoagulation ist im Bereich der Akutdialyse seit Jahren etabliert, die Dosierung optimiert und durch langfristige Studien intensiv überprüft. Üblicherweise wird hierbei durch Citratzugabe im extrakorporalen Blutkreislauf flussaufwärts des Dialysators die koagulierende Wirkung des Blutes durch Binden von Calciumionen in sogenannten Citratcalciumchelaten vermindert. Teilweise werden diese Citratcalciumchelate dem Patienten bei der Blutrückgabe reinfundiert, wo die Citratanteile in der Leber metabolisiert werden und das Calcium wieder frei wird. Ein anderer Teil der Chelate wird über die Dialysatormembran aus dem extrakorporalen Blutkreislauf entfernt und verworfen.

Da der Patient durch diesen Vorgang eine signifikante Calciummenge verliert, kann diese durch künstliche Zugabe von Calcium vor der Blutreinfusion ersetzt werden. Die Zugaberate des Citrats ist üblicherweise an den Blutfluss gekoppelt, um für die entsprechende Menge an Blut, welche mit den Komponenten des extrakorporalen Blutkreislaufs in Berührung kommt, eine adäquate Antikoagulation zur Verfügung zu stellen. Die Zugaberate des Calciums kann so gewählt sein, dass die Calciumverluste über die Dialysatormembran gerade ausgeglichen werden. Sie ist also abhängig vom Blutfluss durch den Dialysator, aber auch von multiplen anderen Parametern, wie beispielsweise von der Citratzugaberate sowie von behandlungsindividuellen Eigenschaften wie beispielsweise von der Wahl der Dialysatormembran, vom jeweils herrschenden Transmembrandruck u.a.

Die Konzentration der Calciumionen kann deshalb zum einen während der CiCa-CRRT regelmäßig durch Probennahme überwacht werden und die Zugaberate kann entsprechend korrigiert werden. Zum anderen können aber auch die Erfahrungen aus den oben genannten Studien genutzt werden, welche in Standardprotokollen zur CiCa-Dosierung verarbeitet wurden, die den Anwendern vorliegen. Dazu kann eine Steuerungsvorrichtung vorgesehen sein, die eine entsprechende Zugabe der antikoagulierenden Substanz steuert. Diese Steuerung kann beispielsweise auf der Basis wenigstens einer oder mehrerer der oben beschriebenen Größen Calciumionenkonzentration, Blutfluss, Dialysatormembran, Transmembrandruck und/oder in der Vorrichtung hinterlegten Standardprotokollen erfolgen.

Wird in einem gemeinsamen extrakorporalen Blutkreislauf zur Akutdialyse eine weitere extrakorporale Blutbehandlung hinzugefügt, können sich ganz neue Randbedingungen bezüglich der Citrat- und Calciumdosierung bei der CiCa-Antikoagulation ergeben. Insbesondere können die aus langfristigen Studienergebnissen etablierten Dosierungsprotokolle bei einer Serienschaltung von Dialysator und Blutbehandlungselement für die weitere extrakorporale Therapie nicht notwendigerweise übernommen werden, da weder der Einfluss des Blutbehandlungselements auf Anreize zur Koagelbildung noch etwaige Calciumverluste über die im Dialysator bekannten hinaus darin berücksichtigt sind.

Die erfindungsgemäße Leitungsführung sieht optional die Anordnung eines Zugabeports zur Zugabe einer ersten medizinischen Flüssigkeit zur Antikoagulation, beispielsweise Citrat, in einem Abschnitt der Hauptblutleitung stromauf des Dialysators vor, dessen gesamter Blutfluss danach auch den Dialysator passieren wird. Damit können die bekannten Dosierungsprotokolle zur CiCa-Antikoagulation trotz Kombinationstherapie weiterhin benutzt werden, da zunächst nur die Koagulation durch den Dialysator berücksichtigt werden muss. Die Komponenten des Blutbehandlungselements können durch Beschichtung antikoagulierend behandelt sein.

Ferner sieht die Leitungsführung optional die Anordnung eines Zugabeports zur Zugabe einer zweiten medizinischen Flüssigkeit zur Antikoagulatoin, beispielsweise Calcium, in der Hauptblutleitung stromab des Blutbehandlungselements vor.

In weiteren Ausführungen der Erfindung, bei denen die erste Verzweigung stromauf des Dialysators angeordnet ist und damit der erste Zugabeport für die erste medizinische Flüssigkeit zur Antikoagulation stromab der ersten Verzweigung liegt, kann ein weiterer Zugabeport für eine dritte medizinische Flüssigkeit zur Antikoagulation, beispielsweise Citrat, an der Hauptblutleitung stromauf der ersten Verzweigung angeordnet sein. Durch zusätzliche Zugabe von Citrat, vorzugsweise von geringen Mengen von Citrat, durch diese Leitung kann im Bedarfsfall kurzfristig eine verstärkte antikoagulierende Wirkung im gesamten extrakorporalen Blutkreislauf erzeugt werden. Die zusätzliche Zugabe über den Zugabeport stromauf der ersten Verzweigung erfolgt vorzugsweise für geringe Mengen von Citrat, da die maximal tolerierte Citratmenge metabolisch begrenzt ist und die größere Wirkung an dem in der Regel nicht antikoagulierend beschichteten Dialysator benötigt wird. Deshalb ist es nicht erforderlich bei der Calciumdosierung in diesem Fall vom bekannten Algorithmus abzuweichen, selbst wenn eine zusätzliche Zugabe von geringen Mengen Citrat durch diese Leitung erfolgt.

Die erste und/oder zweite und/oder dritte medizinische Flüssigkeit zur Antikoagulation kann jeweils auch Heparin oder eine andere medizinische Flüssigkeit mit antikoagulierender Wirkung sein.

In einzelnen oder allen der zuvor genannten Zugabeports, beispielsweise für Calcium oder Citrat, können jeweils Pumpabschnitte für Infusionspumpen angeordnet sein, mit welchen jeweils die zuzugebende medizinische Flüssigkeit aus einem Reservoir durch die Zugabeleitung zur Hauptblutleitung gefördert werden kann. In den Ausführungsformen, in denen dieselbe medizinische Flüssigkeit durch mehrere Zugabeleitungen gefördert wird, insbesondere im Falle von Citrat, kann auch mit einer gemeinsamen Pumpe und/oder aus einem gemeinsamen Reservoir gefördert werden.

Zur Bestimmung des Transmembrandrucks kann die Blutbehandlungsvorrichtung, wie zuvor beschrieben, einen Drucksensor aufweisen, insbesondere zur Messung des Drucks in der Hauptblutleitung zwischen dem Dialysator und dem Blutbehandlungselement, wo in einigen Ausführungsformen der Blutführungsvorrichtung ein entsprechender Druckmessabschnitt für das Abgreifen des Drucks mittels des Drucksensors angeordnet sein kann.

Die Blutbehandlungsvorrichtung kann, wie zuvor beschrieben, zur Bestimmung des Transmembrandrucks einen weiteren Drucksensor aufweisen, insbesondere zur Messung des Drucks in der Hauptblutleitung zwischen der ersten Verzweigung und dem Dialysator. An der benannten Stelle kann in der Blutführungsvorrichtung ein entsprechender Druckmessabschnitt für das Abgreifen des Drucks mittels des Drucksensors angeordnet sein.

Es können auch zwei oder mehr Drucksensoren zur Messung des Drucks an beiden zuvor genannten Stellen oder darüber hinaus noch an anderen Stellen vorgesehen sein. Dies ermöglicht eine besonders genaue Bestimmung des Transmembrandrucks sowie eine bessere Überwachung der Behandlungsverfahren über Grenzwertfenster der jeweiligen Druckwerte.

Für die Messung des Transmembrandrucks können ein oder mehrere Drucksensoren dialysatseitig angeordnet sein. Damit kann der Druck stromauf und/oder stromab des Dialysators auf der Dialysatseite messbar sein.

Um die Kombinationstherapie auf der Seite der Nierenersatztherapien auch mit den Verfahren der Hämofiltration oder Hämodiafiltration zu ermöglichen, kann die Blutführungsvorrichtung ferner optional einen oder mehrere Zugabeports für eine Dilutionsflüssigkeit an der Hauptblutleitung aufweisen. Durch diese Zugabeports kann jeweils mittels entsprechender Infusionspumpen auf der Seite der Blutbehandlungsvorrichtung Dilutionsflüssigkeit, beispielsweise eine Substituatlösung oder Dialyselösung, in die Hauptblutleitung gefördert werden. Optional kann die Erfindung zur Aufbewahrung der Dilutionsflüssigkeit ein oder mehrere Reservoirs aufweisen, z.B. Einwegbeutel. Alternativ kann die Blutbehandlungsvorrichtung eingerichtet sein, die Substituatlösung bzw. Dialyselösung herzustellen. Dazu kann die Blutbehandlungsvorrichtung eine Wasseraufbereitungsvorrichtung mit beispielsweise einer Entgasungsvorrichtung und Konzentratports zur Konnektierung von Konzentratquellen aufweisen. Ein Zugabeport für eine Dilutionsflüssigkeit kann zur Prädilution an der Hauptblutleitung stromauf des Dialysators angeordnet sein. Zur Postdilution kann ein Zugabeport für eine Dilutionsflüssigkeit an der Hauptleitung stromab des Dialysators sowie stromauf des Blutbehandlungselements angeordnet sein. Bei der erfindungsgemäßen Kombinationstherapie mit Dialysator und Blutbehandlungselement ergibt sich noch eine weitere Möglichkeit zur Postdilution. Ein Zugabeport für eine Dilutionsflüssigkeit zur Postdilution kann auch in der Hauptblutleitung stromab des Blutbehandlungselements anordnet sein. Das Einleiten des üblicherweise Calcium-haltigen Substituats stromab des Blutbehandlungselements hat den Vorteil, dass die antikoagulierende Wirkung des Citrats in möglichst weiten Teilen des extrakorporalen Blutkreislaufs eintritt.

Substituatleitungen können auch vom Nutzer wahlweise an eine oder an mehreren der genannten Zugabeports für die Dilutionsflüssigkeit anschließbar sein. Die Dilutionsflüssigkeit kann auch aus einem gemeinsamen Reservoir und/oder mit einer gemeinsamen Infusionspumpe gefördert werden.

Die vorliegende Erfindung wird im Folgenden anhand von Ausführungsbeispielen sowie Zeichnungen näher beschrieben.

Dabei zeigen:
- Figur 1:: eine schematische Darstellung einer Ausführungsform eines erfindungsgemäßen Blutbehandlungssystems.
- Figur 2:: eine schematische Darstellung einer Ausführungsform einer erfindungsgemäßen Blutführungsvorrichtung, mit konnektiertem Dialysator sowie mit konnektiertem Blutbehandlungselement.
- Figur 3a:: in schematischer Darstellung den Flussplan einer Blutführungsvorrichtung.
- Figur 3b:: in schematischer Darstellung den Flussplan der in Figur 3a gezeigten Blutführungsvorrichtung mit weiteren optionalen Komponenten.
- Figur 4a:: in schematischer Darstellung des Flussplans beispielhaft eine alternative Ausführungsvariante der Pumpanordnung, repräsentiert durch die Pumpanordnungsabschnitte.
- Figur 4b:: in schematischer Darstellung des Flussplans beispielhaft eine weitere alternative Ausführungsvariante der Pumpanordnung, repräsentiert durch die Pumpanordnungsabschnitte.
- Figur 4c:: in schematischer Darstellung des Flussplans beispielhaft eine weitere alternative Ausführungsvariante der Pumpanordnung, repräsentiert durch die Pumpanordnungsabschnitte.
- Figur 4d:: in schematischer Darstellung des Flussplans beispielhaft eine weitere alternative Ausführungsvariante der Pumpanordnung, repräsentiert durch die Pumpanordnungsabschnitte.
- Figur 5a:: in schematischer Darstellung den Flussplan einer Blutführungsvorrichtung in einer alternativen Ausführung.
- Figur 5b:: in schematischer Darstellung den Flussplan der in Figur 5a gezeigten Blutführungsvorrichtung mit weiteren optionalen Komponenten.
- Figur 6a:: in schematischer Darstellung den Flussplan einer weiteren alternativen Ausführung einer erfindungsgemäßen Blutführungsvorrichtung.
- Figur 6b:: in schematischer Darstellung den Flussplan der in Figur 6a gezeigten Blutführungsvorrichtung mit alternativer Anordnung des Rezirkulationsrückgabeports.
- Figur 7:: in schematischer Darstellung den Flussplan der in Figur 6a gezeigten Blutführungsvorrichtung mit weiteren optionalen Komponenten.

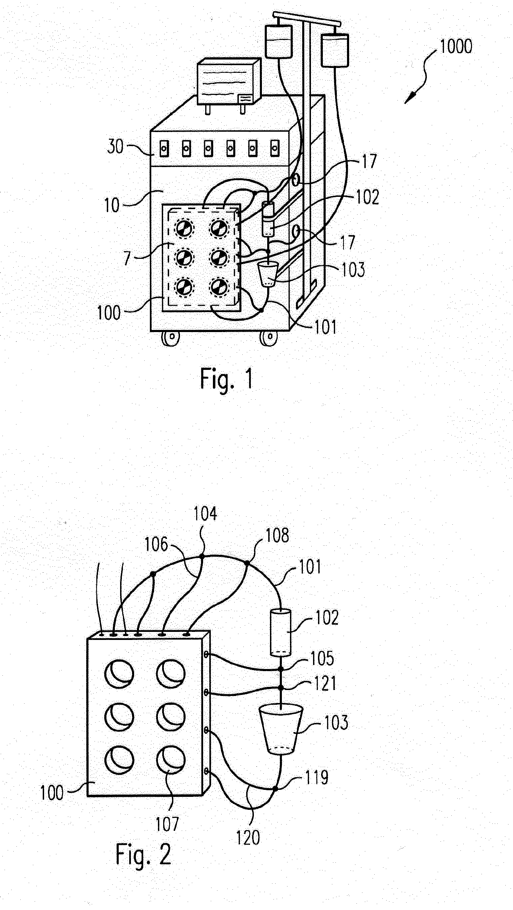

Das Blutbehandlungssystem 1000 weist, wie in Figur 1 dargestellt, eine Blutbehandlungsvorrichtung 10 auf sowie eine Blutführungsvorrichtung 100. In Figur 1 ist die Blutbehandlungsvorrichtung 10 in Form einer Dialysemaschine für die Akutdialyse dargestellt, welche mit einer als Kassette ausgestaltete Blutführungsvorrichtung 100 aufgerüstet ist. Die Blutbehandlungsvorrichtung weist eine Steuerungsvorrichtung 30 auf, sowie eine Pumpanordnung 7. Weiterhin zeigt Figur 1 die Hauptblutleitung 101 der Blutführungsvorrichtung 100, an welche ein Dialysator 102 sowie eine Blutbehandlungselement 103 in Form eines Gasaustauschers angeschlossen ist. Die Hauptblutleitung 101 kann ferner ein oder mehrere Druckmessabschnitte aufweisen, an welchen mittels der optional in der Blutbehandlungsvorrichtung 10 vorhandenen Drucksensoren 17 der Druck abgegriffen werden kann.

Erfindungsgemäß kann die Blutführungsvorrichtung 100 als medizinischer Einwegartikel in Form einer Blutkassette (Figur 2) ausgestaltet sein. Die Blutführungsvorrichtung 100 kann ein oder mehrere Pumpanordnungsabschnitte 107 aufweisen, an denen die Pumpanordnung 7 der Blutbehandlungsvorrichtung 10 einwirken kann, um die Flüssigkeit in dem entsprechenden Leitungsabschnitt der Blutführungsvorrichtung 100 zu fördern. Figur 2 zeigt die Hauptblutleitung 101, welche vom Kassettenkörper der Blutführungsvorrichtung 100 abführt, den Dialysator 102 sowie das Blutbehandlungselement 103, hier in Form eines Gasaustauschers, in Serie aufweist und dann in den Kassettenkörper zurückführt, wo die Pumpanordnungsabschnitte 107 angeordnet sein können. Stromauf des Dialysators 102 kann die erste Verzweigung 104 angeordnet sein, von der die erste Nebenleitung 106 abführt. Stromab des Dialysators 102 sowie stromauf des Blutbehandlungselements 103 kann die zweite Verzweigung 105 angeordnet sein, an welcher sich die erste Nebenleitung 106 wieder mit der Hauptblutleitung 101 vereint. Stromauf des Dialysators 102 kann ferner ein Zugabeport 108 für eine erste medizinische Flüssigkeit zur Antikoagulation, beispielsweise Citrat, angeordnet sein. Stromab des Blutbehandlungselements 103 kann eine Rezirkulationsverzweigung 119 angeordnet sein, von welcher die zweite Nebenleitung 120 abgeführt wird. Die zweite Nebenleitung 120 mündet in einem Rezirkulationsrückgabeport 121, welcher in der Hauptblutleitung 101 stromauf des Blutbehandlungselements 103 sowie stromab des Dialysators 102 angeordnet sein kann.

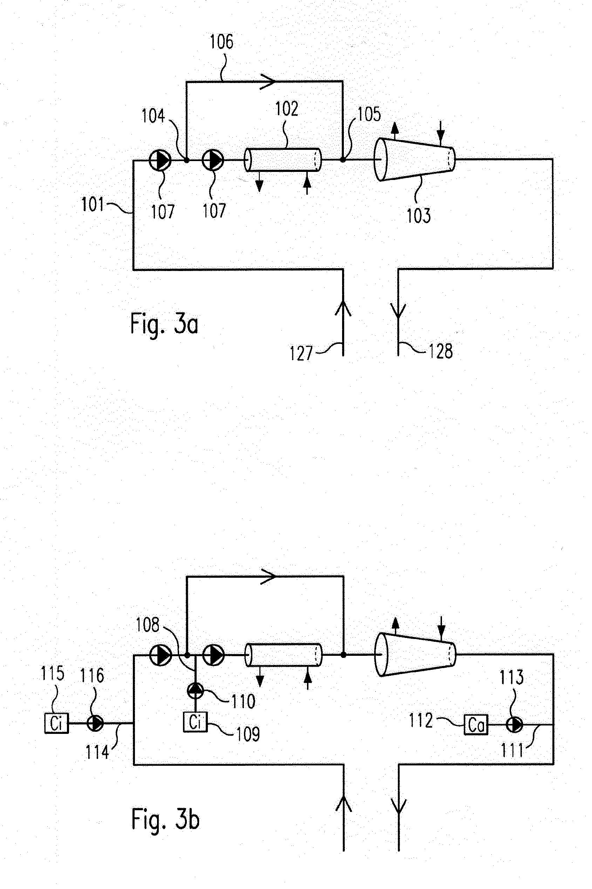

Die Blutführungsvorrichtung 100 weist eine Hauptblutleitung 101 auf, wobei die Hauptblutleitung 101 an einem Ende einem Blutentnahmeport 127 zur Konnektion mit einem Blutentnahmezugang eines Patienten und an einem anderen Ende einen Blutrückgabeport 128 zur Konnektion mit einem Blutrückgabezugang des Patienten aufweist. In der Hauptblutleitung 101 kann das, dem Patienten entnommene Blut, extrakorporal zu dem Dialysator 102 und dem Blutbehandlungselement 103 für die weitere extrakorporale Blutbehandlungstherapie geleitet werden, sowie wieder zurück in den Patienten infundiert werden, wie der Flussplan der Figur 3a schematisch darstellt.

In der Hauptblutleitung ist ein Dialysator 102 angeordnet. Dieser weist üblicherweise eine Blutkammer und eine Dialysatkammer auf (hier nicht dargestellt), wobei die beiden Kammern durch eine semipermeable Membran getrennt sind, über welche das Blut mit der im Dialysatkreislauf fließenden Dialyselösung osmotisch wechselwirken kann. Erfindungsgemäß kann der Dialysator auch für andere in der Dialyse übliche Nierenersatz bzw. Nierenunterstützungstherapien verwendet werden, wie beispielsweise Hämodiafiltration, Hämodialyse, Hämoperfusion, Hämofiltration, ISO-UF usw., insbesondere auch für Therapieverfahren, bei denen keine Dialyselösung auf der Dialysatseite gefördert wird.

Flussabwärts vom Dialysator 102 führt die Hauptblutleitung 101 durch ein Blutbehandlungselement 103, hier als Gasaustauscher ausgestaltet. Dieser weist eine Blutkammer und eine Gaskammer auf (hier nicht dargestellt), wobei die beiden Kammern durch eine semipermeable Membran getrennt sind, über welche das Blut mit dem in der Gasleitung strömenden Gas osmotisch wechselwirken kann.

Sowohl Dialysator 102 als auch Gasaustauscher 103 können dabei eine Vielzahl von einzelnen Membranen in Form von Hohlfasern aufweisen. Dabei können die einzelnen Kammern für Blut, Dialysat oder Gas im Sinne der vorliegenden Erfindung jeweils auch aus einer Vielzahl von innen in den Hohlfasern liegenden Einzelvolumen bestehen, welche am Ende der Fasern fluidisch miteinander in Verbindung stehen.

Stromauf des Dialysators 102 weist die Hauptblutleitung 101 eine erste Verzweigung 104 auf. Von dieser ersten Verzweigung 104 führt eine erste Nebenleitung 106 zu einer zweiten Verzweigung 105 der Hauptblutleitung 101.

Ferner weist die Blutführungsvorrichtung Pumpanordnungsabschnitte 107 auf, an welchen das Blut mittels der Pumpanordnung 7 der Blutbehandlungsvorrichtung 10 sowohl durch die Hauptblutleitung 101 als auch durch die erste Nebenleitung 106 gefördert werden kann. Im Ausführungsbeispiel der Figur 3a ist die erste Pumpanordnung 7 in Form von zwei okkludierenden Blutpumpen ausgeführt, wobei eine in der Hauptblutleitung 101 stromauf von der ersten Verzeigung 104 angeordnet ist und die zweite in der Hauptblutleitung 101 stromab der ersten Verzweigung 104 sowie stromauf des Dialysators 102 angeordnet ist. In diesem Ausführungsbeispiel pumpt die erste Pumpe beispielsweise mit 500 ml/min, während die zweite Pumpe nur mit 200 ml/min fördert. Damit stellt sich in der Nebenleitung 106 ein Fluss von 300 ml/min ein. Am Dialysator 102 stellt sich ein Blutfluss von 200 ml/min ein und der Gasaustauscher 103 wird mit 500 ml/min durchströmt, da sich die beiden Teilflüsse aus 200 ml/min und 300 ml/min an der zweiten Verzweigung 105 wieder vereinigen.

Wie in Figur 3b schematisch gezeigt, kann die Blutführungsvorrichtung optional Zugabeports für medizinische Flüssigkeit zur Antikoagulation aufweisen. So kann stromab der ersten Verzweigung 104 sowie stromauf des Dialysators 102 ein Zugabeport 108 für Citratlösung angeordnet sein. Dieser kann auch als Citratleitung 108 ausgestaltet sein, welche mit einem Citratreservoir 109 verbunden ist. Ferner kann die Blutbehandlungsvorrichtung 10 eine Infusionspumpe 110 aufweisen, welche ausgebildet ist, um Citrat aus dem Reservoir 112 über den Zugabeport 108 in die Hauptblutleitung 101 zur fördern. Stromab des Blutbehandlungselements 103 kann ein Zugabeport 111 für Calciumlösung angeordnet sein. Dieser kann auch als Calciumleitung 111 ausgestaltet sein, welche mit einem Calciumreservoir 112 verbunden ist. Ferner kann die Blutbehandlungsvorrichtung 10 eine weitere Infusionspumpe 113 aufweisen, welche ausgebildet ist, um Calcium aus dem Reservoir 112 über den Zugabeport 111 in die Hauptblutleitung 101 zu fördern.

Figur 3b zeigt ferner eine weitere, optionale Citratzugabemöglichkeit. Hierzu ist stromauf der ersten Verzeigung 104 in der Hauptblutleitung ein dritter Zugabeport 114 für medizinische Flüssigkeit zur Antikoagulation angeordnet. Der Zugabeport 114 kann auch als Zugabeleitung ausgestaltet sein, welche mit einem Citratreservoir 115 verbunden sein kann. Ferner kann die Blutbehandlungsvorrichtung 10 eine weitere Infusionspumpe 116 aufweisen, welche ausgebildet ist, um Citrat aus dem Reservoir 115 über den Zugabeport 114 in die Hauptblutleitung 101 zu fördern. Alternativ (hier nicht gezeigt) kann die dritte Zugabeleitung 114 auch aus dem ersten Reservoir 109 gespeist werden. Weiterhin alternativ kann auch die dritte Infusionspumpe 116 entfallen, wenn der von der ersten Infusionspumpe 110 erzeugte Druck in einer danach abzweigenden dritten Zugabeleitung genutzt wird. Hierzu kann die dritte Zugabeleitung ein Ventil oder eine Drossel aufweisen, die den Druck entsprechend einstellt.

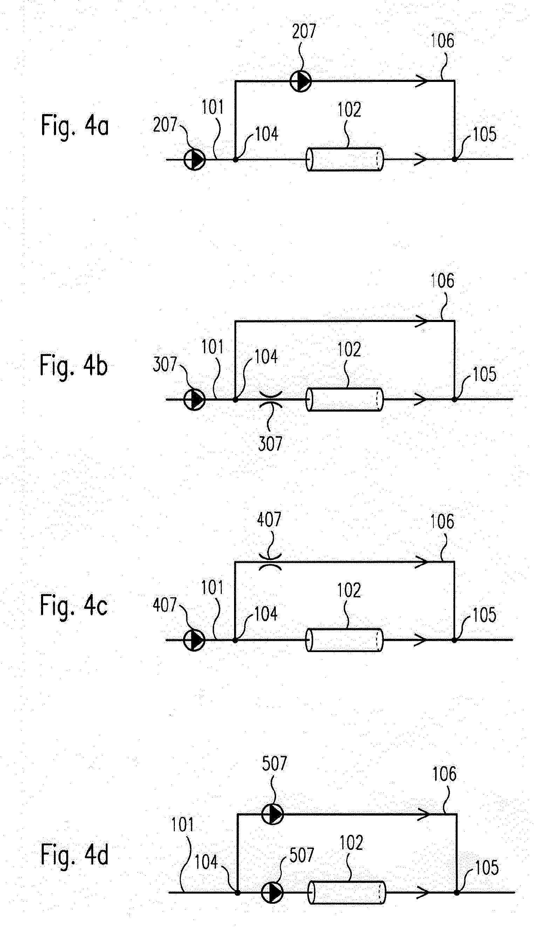

Die Figuren 4a bis 4d zeigen schematisch jeweils einen Ausschnitt der Hauptblutleitung 101 der Blutführungsvorrichtung 100 im Bereich um die erste Verzweigung 104 und die zweite Verzweigung 105, welcher in dieser Ausführung die beiden Flusswege über die Nebenleitung 106 sowie über den Abschnitt der Hauptblutleitung 101 durch den Dialysator 102 enthält. Die Figuren 4a bis 4d zeigen beispielhaft verschiedene mögliche Ausführungen der Pumpanordnung, repräsentiert durch die Pumpanordnungsabschnitte 207, 307, 407, 507 zur Einwirkung der Pumpanordnung 7 der Blutbehandlungsvorrichtung 10.

Während die Pumpanordnung der in Figur 3a genannten Ausführung, repräsentiert durch die Pumpanordnungsabschnitte 107, eine okkludierende Blutpumpe in der Hauptblutleitung 101 flussaufwärts der ersten Verzweigung 104 und eine weitere okkludierende Blutpumpe in der Hauptblutleitung 101 zwischen Dialysator 102 und der ersten Verzweigung 104 aufweist, zeigt Figur 4a eine Variante der Pumpanordnung, repräsentiert durch die Pumpanordnungsabschnitte 207, welche ebenfalls eine erste Blutpumpe in der Hauptblutleitung 101 flussaufwärts der ersten Verzweigung 104 aufweist, jedoch mit einer alternativen Anordnung der weiteren okkludierenden Blutpumpe in der Nebenleitung 106.

Bei den, in den Figuren 4b und 4c gezeigten Beispielen von Pumpanordnungen, repräsentiert durch die Pumpanordnungsabschnitte 307, 407, sind die weiteren Blutpumpen in der Nebenleitung 106 bzw. in der Hauptblutleitung 101 zwischen Dialysator 102 und der ersten Verzweigung 104 jeweils durch Drosselelemente ersetzt.

Die Figur 4d zeigt eine weitere beispielhafte Ausführung einer ersten Pumpanordnung, repräsentiert durch die Pumpanordnungsabschnitte 507, welche zwei okkludierende Blutpumpen aufweist, wobei eine in der Nebenleitung 106 angeordnet ist und die andere sich in der Hauptblutleitung 101 zwischen Dialysator 102 und der ersten Verzweigung 104 befindet.

Alle erfindungsgemäßen Pumpanordnungen können sowohl einen Blutfluss in der Hauptblutleitung 101 erzeugen als auch einen Teilfluss mit definierter Flussrate über die Nebenleitung 106 führen, sodass damit das Verhältnis zwischen dem gesamten Fluss in der Hauptblutleitung 101 flussaufwärts der ersten Verzeigung 104 bzw. flussabwärts der zweiten Verzweigung 105 und im Bereich des Dialysators 102 einstellbar ist. Wie der Fachmann erkennt, existieren im Stand der Technik noch zahlreiche weitere Möglichkeiten, das Verhältnis der Flüsse in den beiden Leitungsabschnitten zu steuern. In manchen Ausführungsformen kann die Pumpanordnung 7 die Flüsse der beiden Leitungsabschnitte unabhängig voneinander steuern. Die Pumpanordnung 7 kann verschiedene fluidische Komponenten aufweisen, einschließlich okkludierender Pumpen, nichtokkludierender Pumpen, Klemmen, Ventile, Drosseln etc. Die Komponenten der Pumpanordnung 7 können an anderen Stellen im extrakorporalen Blutkreislauf angeordnet sein oder einwirken.

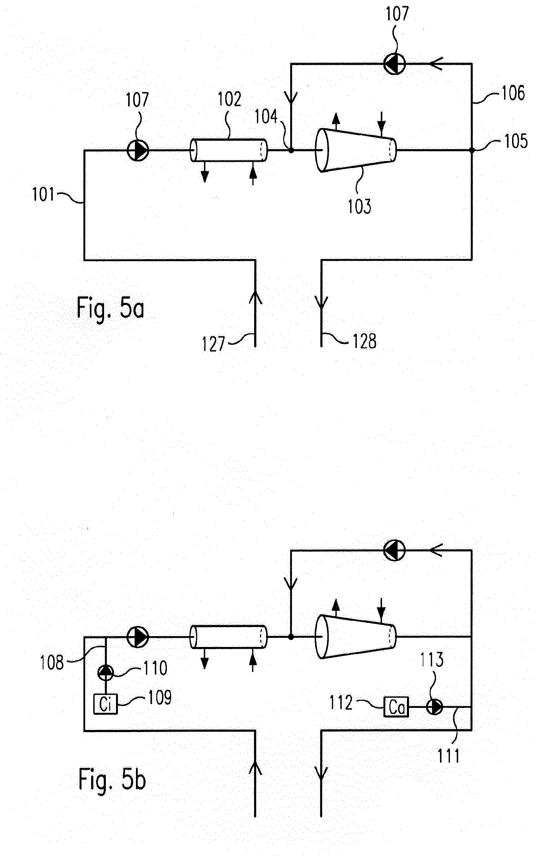

Wie in Figur 5a schematisch anhand des Flussplanes gezeigt, kann die Blutführungsvorrichtung 100 auch eine alternative Flussführung aufweisen. In diesem Beispiel ist die erste Verzweigung 104, an welcher die Nebenleitung 106 von der Hauptblutleitung 101 abzweigt, stromab des Blutbehandlungselements 103 angeordnet. Ferner ist in diesem Beispiel die zweite Verzweigung 105, an welcher sich die Nebenleitung 106 wieder mit der Hauptblutleitung 101 vereinigt, stromauf des Blutbehandlungselements 103 sowie stromab des Dialysators 102 angeordnet.

Wie in Figur 5b schematisch gezeigt, kann die Blutführungsvorrichtung optional Zugabeports für medizinische Flüssigkeit zur Antikoagulation aufweisen. So kann stromauf des Dialysators 102 ein Zugabeport 108 für Citratlösung angeordnet sein. Dieser kann auch als Citratleitung 108 ausgestaltet sein, welche mit einem Citratreservoir 109 verbunden ist. Ferner kann die Blutbehandlungsvorrichtung 10 eine Infusionspumpe 110 aufweisen, welche ausgebildet ist, um Citrat aus dem Reservoir 112 über den Zugabeport 108 in die Hauptblutleitung 101 zur fördern. Stromab des Blutbehandlungselements 103 kann ein Zugabeport 111 für Calciumlösung angeordnet sein. Dieser kann auch als Calciumleitung 111 ausgestaltet sein, welche mit einem Calciumreservoir 112 verbunden ist. Ferner kann die Blutbehandlungsvorrichtung 10 eine weitere Infusionspumpe 113 aufweisen, welche ausgebildet ist, um Calcium aus dem Reservoir 112 über den Zugabeport 111 in die Hauptblutleitung 101 zu fördern.

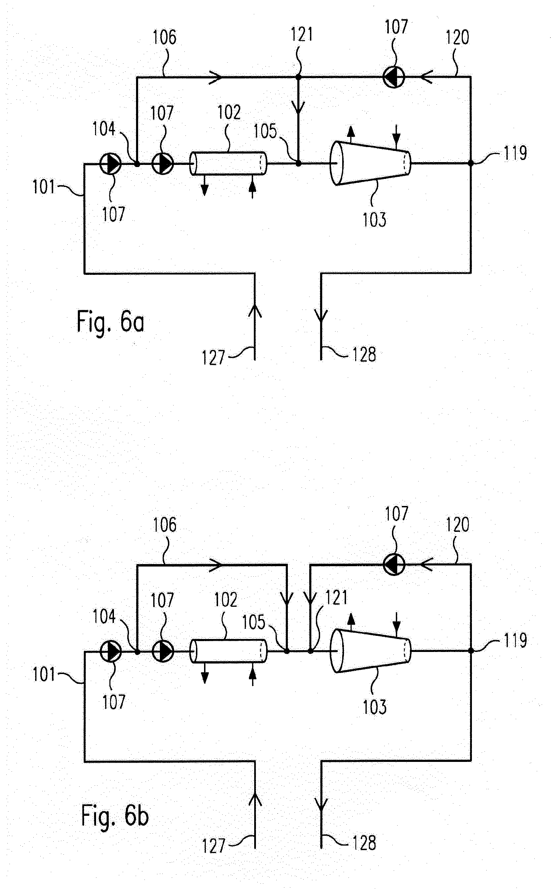

Figur 6a zeigt schematisch anhand des Flussplans eine Ausführung einer erfindungsgemäßen Blutführungsvorrichtung, wobei hier zusätzlich zu den Merkmalen der Figur 3a eine zweite Nebenleitung 120 für die mehrmalige Rezirkulation des Bluts durch das, hier als Gasaustauscher ausgestaltete Blutbehandlungselement 103 vorliegt. Die zweite Nebenleitung 120 führt an einer Rezirkulationsverzweigung 119 von der Hauptblutleitung 101 ab und mündet in einem Rezirkulationsrückgabeport 121. Im Beispiel der Figur 6a ist der Rezirkulationsrückgabeport 121 in der ersten Nebenleitung 106 stromauf der zweiten Verzweigung 105 angeordnet. Das Beispiel der Figur 6b zeigt hierzu eine alternative Leitungsführung und unterscheidet sich von dem in Figur 6a gezeigten darin, dass der Rezirkulationsrückgabeport 121 direkt in der Hauptblutleitung 101 stromab des Dialysators 102 sowie stromauf des Blutbehandlungselements 103 angeordnet ist. In den Ausführungen der Figuren 6a und 6b kann die Pumpanordnung 7 der Blutbehandlungsvorrichtung 10 ferner zur Erzeugung eines Blutflusses in der zweiten Nebenleitung 120 eingerichtet sein. Hierzu umfasst die Pumpanordnung 7 der Blutbehandlungsvorrichtung 10 in den Figuren 6a und 6b - hier repräsentiert durch die Pumpanordnungsabschnitte 107 - eine weitere okkludierende Pumpe in der zweiten Nebenleitung 120. Darüber hinaus kann die Steuerungsvorrichtung 30 der Blutbehandlungsvorrichtung 10 dazu konfiguriert sein, die Pumpanordnung 7 derart zu betreiben, dass die Blutflussrate in mindestens einem Abschnitt der Hauptblutleitung 101 zumindest von einer der Blutflussraten in den Nebenleitungen 106, 120 unabhängig ist.

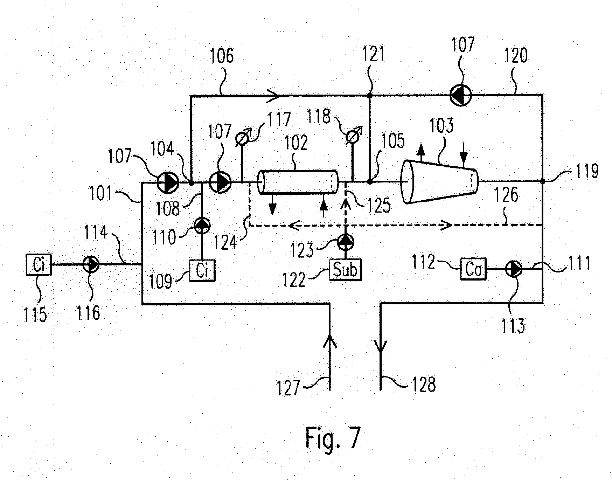

Figur 7 zeigt schematisch eine Ausführung der Blutführungsvorrichtung aus Figur 6a mit zusätzlichen optionalen Komponenten. Auch in der Ausführung mit zwei Nebenleitung 106, 120 können die, bereits zum Ausführungsbeispiel von Figur 3b beschriebenen Zugabeports 108, 111, 114, die entsprechenden Infusionspumpen 110, 113, 116 sowie Reservoirs 109, 112, 115 vorgesehen sein.

Figur 7 zeigt weiterhin zwei optionale Druckmessungen 117,118 in der Hauptblutleitung 101 stromab sowie stromauf des Dialysators 102. Zum einen ist der Druck stromab des Dialysators und optional auch stromauf des Dialysators hilfreich, um den Transmembrandruck zu bestimmen. Dieser stellt eine wichtige Größe dar, welche im Verlauf der Dialysetherapie beispielsweise Auskunft über eine drohende Filterverstopfung gibt. Der Transmembrandruck kann auch bei der Festlegung der Calciumzugaberate über den zweiten Zugabeport 111 berücksichtigt werden. Zum anderen sind die Drücke in den jeweiligen Flussabschnitten auch hilfreich um die Therapie mittels Druckgrenzwertfenster zu überwachen.

Weiternhin zeigt die Figur 7 optionale Komponenten, welche auf der Seite der Nierenersatztherapie auch Hämofiltration und/oder Hämodiafiltration erlauben. Hierzu kann der extrakorporale Blutkreislauf eine oder mehrere Zugabeports 124,125,126 für Dilutionsflüssigkeit, optional auch als Substituatleitung ausgestaltet, aufweisen, durch welche aus einem Reservoir 122 Substitutionsflüssigkeit mittels einer weiteren Infusionspumpe 123 in die Hauptblutleitung 101 zugegeben werden kann. Die Substituatleitung kann dabei in Prädilution 124 angeschlossen sein, dann mündet sie stromauf des Dialysators 102 in die Hauptblutleitung 101. Die Substituatleitung kann auch in Postdilution angeschlossen sein. Bei der Postdilution bietet der erfindungsgemäße Blutkreislauf zwei mögliche Anschlusspositionen. In einer ersten Option kann die Postdilutionsleitung 125 zwischen Dialysator 102 und Blutbehandlungselement 103 in die Hauptblutleitung 101 münden. In einer zweiten Option kann die Postdilutionsleitung 126 auch stromab des Gasaustauschers in die Hauptblutleitung 101 münden. Die letzte Variante 126 bietet dabei den Vorteil, dass die Substitutionslösung, welche üblicherweise Calcium enthält, die antikoagulierende Wirkung des Citrats erst im hinteren Teil des extrakorporalen Blutkreislaufs vermindert.

Alternativ kann die Substituatleitung auch wahlweise vom Nutzer an einer oder mehreren der genannten Positionen angeschlossen werden. In einer Kombination aus Prä- und Postdilution sind wahlweise auch zwei unabhängig voneinander fördernde Infusionspumpen für die Dilutionsflüssigkeit möglich (nicht dargestellt).

Die Blutbehandlungsvorrichtung 10 umfasst eine Steuerungsvorrichtung 30. Die Steuerungsvorrichtung 30 kann zur Steuerung und Regelung eines Behandlungsverfahrens konfiguriert sein. Gemäß einem Verfahren kann in allen Ausführungsbeispielen mittels der Pumpanordnung 7 ein Blutfluss in der Hauptblutleitung 101 im Bereich des Dialysators 102 zwischen 0 und 300 ml/min erzeugt werden. Darüber hinaus kann im Bereich des Blutbehandlungselements 103 ein Blutfluss von größer als 500 ml/min erzeugt werden. In Beispiel der Figuren 3a und 3b entspricht die Flussrate in der Hauptblutleitung 101 vor der ersten Verzweigung 104 der Flussrate im Blutbehandlungselement 103. Mittels der Pumpanordnung 7 kann die gewünschte Dialysatorflussrate eingestellt werden. Damit ergibt sich die Flussrate in der Nebenleitung 106 als Differenz der Flussrate im Blutbehandlungselement 103 und der Dialysatorflussrate.

Die Flussrate im Blutbehandlungselement 103 kann in allen Ausführungsformen der Erfindung auch größer als 800 ml/min, größer als 1 l/min oder größer als 2 l/min sein. Üblicherweise werden ECMO-Verfahren mit Blutflüssen von bis zu 8 l/min betrieben. Alle diese Flussraten und sogar darüber hinausgehende sind erfindungsgemäß im Bereich eines, beispielsweise als Gasaustauscher ausgestalteten, Blutbehandlungselements 103 möglich.

Die Dialysatorflussrate kann in allen Ausführungsformen auch im Bereich zwischen 100 und 250 ml/min liegen. Sie kann auch im Bereich 175 bis 225 ml/min liegen oder genau 200 ml/min betragen.

Die Flussrate der ersten medizinischen Flüssigkeit zur Antikoagulation, beispielsweise Citrat, welche mittels der ersten Infusionspumpe 110 in die Hauptblutleitung 101 gefördert wird, kann von der Steuerungsvorrichtung 30 in Abhängigkeit von der Dialysatorflussrate geregelt sein.

Die Flussrate der zweiten medizinischen Flüssigkeit zur Antikoagulation, beispielsweise Calicum, welche mittels der zweiten Infusionspumpe 113 in die Hauptblutleitung 101 gefördert wird, kann von der Steuerungsvorrichtung 30 in Abhängigkeit von der Dialysatorflussrate geregelt sein. Die Regelung kann zusätzlich noch andere Abhängigkeiten berücksichtigen, wie beispielsweise die Flussrate der ersten medizinischen Flüssigkeit zur Antikoagulation, die Flussrate der dritten medizinischen Flüssigkeit zur Antikoagulation, den Transmembrandruck (TMP), die Art des Dialysators und/oder andere, optional auch von Nutzer auszuwählende oder einzugebende Parameter.

Die Flussrate der dritten medizinischen Flüssigkeit zur Antikoagulation, beispielsweise Citrat, kann mittels direkter Auswahl des Nutzers gesteuert sein. Sie kann auch abhängig von der Flussrate im Blutbehandlungselement 103 geregelt sein oder von der Differenz aus der Flussrate im Blutbehandlungselement 103 und der Dialysatorflussrate.

## Patentansprüche

1. Blutführungsvorrichtung (100) zum Zusammenwirken mit einer Blutbehandlungsvorrichtung (10),
aufweisend:
- eine Hauptblutleitung (101) fluidisch konnektiert mit einem Dialysator (102) sowie fluidisch konnektiert mit einem Blutbehandlungselement (103) stromab des Dialysators (102), wobei die Hauptblutleitung (101) an einem Ende einen Blutentnahmeport (127) zur Konnektion mit einem Blutentnahmezugang eines Patienten und an einem anderen Ende einen Blutrückgabeport (128) zur Konnektion mit einem Blutrückgabezugang des Patienten aufweist;
- mindestens eine Nebenleitung (106), welche an einer ersten Verzweigung (104) von der Hauptblutleitung (101) abführt und sich an einer zweiten Verzweigung (105) wieder mit der Hauptblutleitung (101) vereinigt;
und
- einen oder mehrere Pumpanordnungsabschnitte (107), ausgebildet zum Einwirken der Pumpanordnung (7) der Blutbehandlungsvorrichtung (10), **dadurch gekennzeichnet, dass**
die Blutführungsvorrichtung (100) weiterhin eine zweite Nebenleitung (120) aufweist, welche an einer Rezirkulationsverzweigung (119) von der Hauptblutleitung (101) abführt und in einem Rezirkulationsrückgabeport (121) mündet,
wobei die erste Verzweigung (104) stromauf der Konnektionsstelle für den Dialysator (102) angeordnet ist,
und
wobei die zweite Verzweigung (105) stromab der Konnektionsstelle für den Dialysator (102) sowie stromauf der Konnektionsstelle für das Blutbehandlungselement (103) angeordnet ist,
und
wobei die Rezirkulationsverzweigung (119) stromab der Konnektionsstelle für das Blutbehandlungselement (103) angeordnet ist,
und
wobei der Rezirkulationsrückgabeport (121) in der Hauptblutleitung (101) stromauf der Konnektionsstelle für das Blutbehandlungselement (103) sowie stromab der Konnektionsstelle für den Dialysator (102) angeordnet ist,
oder
wobei der Rezirkulationsrückgabeport (121) in der ersten Nebenleitung (106) stromauf der zweiten Verzweigung (105) angeordnet ist.

2. Blutführungsvorrichtung (100) nach Anspruch 1,
wobei die Hauptblutleitung (101) aufweist:
- einen Zugabeport (108) für eine erste medizinische Flüssigkeit zur Antikoagulation stromauf der Konnektionsstelle für den Dialysator (102);
und/oder
- einen Zugabeport (111) für eine zweite medizinische Flüssigkeit zur Antikoagulation stromab der Konnektionsstelle für das Behandlungselement (103).

3. Blutführungsvorrichtung (100) nach Anspruch 1,
wobei die Hauptblutleitung (101) aufweist:
- einen Zugabeport (108) für eine erste medizinische Flüssigkeit zur Antikoagulation stromab der ersten Verzweigung (104) sowie stromauf der Konnektionsstelle für den Dialysator (102);
und/oder
- einen Zugabeport (111) für eine zweite medizinische Flüssigkeit zur Antikoagulation stromab der Konnektionsstelle für das Behandlungselement (103);
und/oder
- einen Zugabeport (114) für eine dritte medizinische Flüssigkeit zur Antikoagulation stromauf der ersten Verzweigung (104).

4. Blutführungsvorrichtung (100) nach einem der Ansprüche 1 bis 3,
wobei die Hauptblutleitung (101) einen Druckmessabschnitt (117) für die Bestimmung des Drucks in der Hauptblutleitung (101) stromab der Konnektionsstelle für den Dialysator (102) sowie stromauf der Konnektionsstelle für das Blutbehandlungselement (103) aufweist.

5. Blutführungsvorrichtung (100) nach einem der Ansprüche 1 bis 4,
wobei die Hauptblutleitung (101) aufweist:
- einen Zugabeport (124) für eine Dilutionsflüssigkeit stromauf der Konnektionsstelle für den Dialysator (102);
und/oder
- einen Zugabeport (125) für eine Dilutionsflüssigkeit stromab der Konnektionsstelle für den Dialysator (102) sowie stromauf der Konnektionsstelle für das Blutbehandlungselement (103);
und/oder
- einen Zugabeport (126) für eine Dilutionsflüssigkeit stromab der Konnektionsstelle für das Blutbehandlungselement (103).

6. Blutführungsvorrichtung (100) nach einem der Ansprüche 1 bis 5,
wobei das Blutbehandlungselement (103) ein Gasaustauscher ist.

7. Blutbehandlungssystem (1000) zur Durchführung einer extrakorporalen Blutbehandlung aufweisend eine Blutbehandlungsvorrichtung (10) und eine Blutführungsvorrichtung (100) nach einem der Ansprüche 1 bis 6, wobei die Blutbehandlungsvorrichtung (10) aufweist:
- eine Steuerungsvorrichtung (30);
und
- eine Pumpanordnung (7), welche zur Erzeugung von Blutflüssen in der Hauptblutleitung (101), sowie in der mindestens einen Nebenleitung (106) eingerichtet ist, wobei die Pumpanordnung wenigstens zwei Elemente umfasst, die auf den Fluss in der Hauptblutleitung (101) und in der mindestens einen Nebenleitung (106) der Blutführungsvorrichtung (100) wirken,
wobei die Steuerungsvorrichtung (30) konfiguriert ist, die Pumpanordnung (7) derart zu betreiben, dass eine erste Blutflussrate im Dialysator (102) von einer zweiten Blutflussrate in dem Blutbehandlungselement (103) entkoppelt ist, wobei weiter
- die Pumpanordnung (7) ferner zur Erzeugung eines Blutflusses in der zweiten Nebenleitung (120) eingerichtet ist,
und
- wobei die Steuerungsvorrichtung (30) konfiguriert ist, die Pumpanordnung (7) derart zu betreiben, dass die Blutflussrate in mindestens einem Abschnitt der Hauptblutleitung (101) zumindest von einer der Blutflussraten in den Nebenleitungen (106, 120) unabhängig ist.

8. Blutbehandlungssystem (1000) nach Anspruch 7, wobei die Pumpenanordnung (7) der Blutbehandlungsvorrichtung (10) ausgebildet ist, um in der Hauptblutleitung (101) und in der mindestens einen Nebenleitung (106) der Blutführungsvorrichtung voneinander unabhängige Blutflussraten zu erzeugen.

9. Blutbehandlungssystem (1000) nach einem der Ansprüche 7 oder 8,
wobei die Blutbehandlungsvorrichtung (10) aufweist:
- eine Infusionspumpe (110) zur Zuleitung medizinischer Flüssigkeit in die Hauptblutleitung (101),
oder
- zwei Infusionspumpen (110, 113) zur Zuleitung medizinischer Flüssigkeit in die Hauptblutleitung (101),
oder
- drei Infusionspumpen (110, 113, 116) zur Zuleitung medizinischer Flüssigkeit in die Hauptblutleitung (101),
oder
- vier oder mehr Infusionspumpen (110, 113, 116, 123) zur Zuleitung medizinischer Flüssigkeit in die Hauptblutleitung (101).

10. Blutbehandlungssystem (1000) nach Anspruch 9,
wobei die Steuerungsvorrichtung (30) der Blutbehandlungsvorrichtung (10) konfiguriert ist, um die Zuleitungsrate mindestens einer der Infusionspumpen abhängig von der Blutflussrate im Dialysator (102) zu regeln, insbesondere wobei die Steuerungsvorrichtung (30) konfiguriert ist, um die Zuleitungsraten von mindestens zwei der Infusionspumpen jeweils abhängig von der Blutflussrate im Dialysator (102) zu regeln.

11. Blutbehandlungssystem (1000) nach einem der Ansprüche 7 bis 10,
wobei die Blutbehandlungsvorrichtung (10) einen Drucksensor (17) zur Bestimmung des Drucks in der Hauptblutleitung (101) stromab des Dialysators (102) sowie stromauf des Blutbehandlungselements (103) aufweist.

## Claims

1. A blood guidance device (100) for cooperating with a blood treatment device (10) comprising:
- a main blood line (101) for fluidic connection to a dialyzer (102) and for fluidic connection to a blood treatment element (103) downstream from the dialyzer (102), wherein the main blood line (101) has at one end a blood withdrawal port (127) for connection to a blood withdrawal access of a patient and at the other end has a blood return port (128) for connection to a blood return access of the patient;
- at least one secondary line (106), which branches off from the main blood line (101) at a first branch (104) and recombines with the main blood line (101) at a second branch (105); and
- one or more pump configuration sections (107), designed for action of the pump configuration (7) on the blood treatment device (10),
**characterized in that**
the blood guidance device (100) comprises a second secondary line (120), which branches off from the main blood line (101) at a recirculation branch (119) and opens into a recirculation return port (121),
wherein the first branch (104) is disposed upstream from the connection point for the dialyzer (102), and
wherein the second branch (105) is disposed downstream from the connection point for the dialyzer (102) and upstream from the connection point for the blood treatment element (103), and
wherein the recirculation branch (119) is disposed downstream from the connection point for the blood treatment element (103), and
wherein the recirculation return port (121) is disposed in the main blood line (101) upstream from the connection point for the blood treatment element (103) and downstream from the connection point for the dialyzer (102), or
wherein the recirculation return port (121) is disposed in the first secondary line (106) upstream from the second branch (105).

2. The blood guidance device (100) according to claim 1, wherein the main blood line (101) comprises:
- an injection port (108) for a first medical fluid for anticoagulation upstream from the connection point for the dialyzer (102); and/or
- an injection port (111) for a second medical fluid for anticoagulation downstream from the connection point for the treatment element (103).

3. The blood guidance device (100) according to claim 1, wherein the main blood line (101) comprises:
- an injection port (108) for a first medical fluid for anticoagulation downstream from the first branch (104) and upstream from the connection point for the dialyzer (102);
and/or
- an injection port (111) for a second medical fluid for anticoagulation downstream from the connection point for the treatment element (103);
and/or
- an injection port (114) for a third medical fluid for anticoagulation upstream from the first branch (104).

4. The blood guidance device (100) according to any one of claims 1 to 3, wherein the main blood line (101) has a pressure measurement section (117) for determining the pressure in the main blood line (101) downstream from the connection point for the dialyzer (102) and upstream from the connection point for the blood treatment element (103).

5. The blood guidance device (100) according to any one of claims 1 to 4, wherein the main blood line (101) comprises:
- an injection port (124) for a dilution fluid upstream from the connection point for the dialyzer (102); and/or
- an injection port (125) for a dilution fluid downstream from the connection point for the dialyzer (102) and upstream from the connection point for the blood treatment element (103); and/or
- an injection port (126) for a dilution fluid downstream from the connection point for the blood treatment element (103).

6. The blood guidance device (100) according to any one of claims 1 to 5, wherein the blood treatment element (103) is a gas exchanger.

7. A blood treatment system (1000) for carrying out an extracorporeal blood treatment, comprising a blood treatment device (10) and a blood guidance device (100) according to any one of claims 1 to 6; wherein the blood treatment device (10) has:
- a control device (30); and
- a pump configuration (7), which is equipped for generating blood flows in the main blood line (101) and also in the at least one secondary line (106), wherein the pump configuration comprises at least two elements that act on the flow rate in the main blood line (101) and in the at least one secondary line (106) of the blood guidance device (100),
wherein the control device (30) is configured to operate the pump configuration (7) in such a way that a first blood flow rate in the dialyzer (102) is decoupled from a second blood flow rate in the blood treatment element (103), wherein further
the pump configuration (7) is also equipped for generating a blood flow in the second secondary line (120), and
wherein the control device (30) is configured to operate the pump configuration (7) in such a way that the blood flow rate in at least one section of the main blood line (101) is independent at least of one of the blood flow rates in the secondary lines (106, 120).

8. The blood treatment system (1000) according to claim 7, wherein the pump configuration (7) is designed to generate independent blood flow rates in the main blood line (101) and in the at least one secondary line (106).

9. The blood treatment system (1000) according to claim 7 or 8, wherein the blood treatment device (10) has:
- an infusion pump (110) for supplying medical fluid to the main blood line (101), or
- two infusion pumps (110, 113) for supplying medical fluid into the main blood line (101), or
- three infusion pumps (110, 113, 116) for supplying medical fluid into the main blood line (101), or
- four or more infusion pumps (110, 113, 116, 123) for supplying medical fluid into the main blood line (101).

10. The blood treatment system (1000) according to claim 9, wherein the control device (30) is configured to regulate the delivery rate of at least one of the infusion pumps as a function of the blood rate in the dialyzer (102), in particular wherein the control device (30) is configured to regulate the delivery rates of at least two of the infusion pumps each as a function of the blood flow rate in the dialyzer (102).

11. The blood treatment system (1000) according to any one of claims 7 to 10, wherein the blood treatment device (10) has a pressure sensor (17) for determining the pressure in the main blood line (101) downstream from the dialyzer (102) and upstream from the blood treatment element (103).

## Revendications

1. Dispositif d'administration de sang (100) pour coopérer avec un dispositif de traitement de sang (10),
présentant :
- un conduit de sang principal (101) connecté fluidiquement à un dialyseur (102) ainsi que connecté fluidiquement à un élément de traitement de sang (103) en aval du dialyseur (102), dans lequel le conduit de sang principal (101) comporte, à une extrémité, un orifice de prélèvement de sang (127) à connecter à une entrée de prélèvement de sang d'un patient et, à une autre extrémité, un orifice de retour de sang (128) à connecter à une entrée de retour de sang du patient ;
- au moins un conduit secondaire (106), lequel sur une première ramification (104) s'éloigne du conduit de sang principal (101) et sur une deuxième ramification (105) s'unit à nouveau au conduit de sang principal (101) ;
et
- une ou plusieurs parties d'ensemble de pompe (107), réalisées pour agir sur l'ensemble de pompe (7) du dispositif de traitement de sang (10), **caractérisé en ce que**
le dispositif d'administration de sang (100) comporte en outre un deuxième conduit secondaire (120), lequel sur une ramification de recirculation (119) s'éloigne du conduit de sang principal (101) et débouche dans un orifice de retour de recirculation (121),
dans lequel la première ramification (104) est disposée en amont du point de connexion pour le dialyseur (102),
et
dans lequel la deuxième ramification (105) est disposée en aval du point de connexion pour le dialyseur (102) ainsi qu'en amont du point de connexion pour l'élément de traitement de sang (103),
et
dans lequel la ramification de recirculation (119) est disposée en aval du point de connexion pour l'élément de traitement de sang (103),
et
dans lequel l'orifice de retour de recirculation (121) est disposé dans le conduit de sang principal (101) en amont du point de connexion pour l'élément de traitement de sang (103) ainsi qu'en aval du point de connexion pour le dialyseur (102), ou
dans lequel l'orifice de retour de recirculation (121) est disposé dans le premier conduit secondaire (106) en amont de la deuxième ramification (105).

2. Dispositif d'administration de sang (100) selon la revendication 1,
dans lequel le conduit de sang principal (101) comporte :
- un orifice d'entrée (108) pour un premier liquide médical d'anticoagulation en amont du point de connexion pour le dialyseur (102) ;
et/ou
- un orifice d'entrée (111) pour un deuxième liquide médical d'anticoagulation en aval du point de connexion pour l'élément de traitement (103).

3. Dispositif d'administration de sang (100) selon la revendication 1,
dans lequel le conduit de sang principal (101) comporte :
- un orifice d'entrée (108) pour un premier liquide médical d'anticoagulation en aval de la première ramification (104) ainsi qu'en amont du point de connexion pour le dialyseur (102) ;
et/ou
- un orifice d'entrée (111) pour un deuxième liquide médical d'anticoagulation en aval du point de connexion pour l'élément de traitement (103) ;
et/ou
- un orifice d'entrée (114) pour un troisième liquide médical d'anticoagulation en amont de la première ramification (104) .

4. Dispositif d'administration de sang (100) selon l'une quelconque des revendications 1 à 3,
dans lequel le conduit de sang principal (101) comporte une partie de mesure de pression (117) pour la détermination de la pression dans le conduit de sang principal (101) en aval du point de connexion pour le dialyseur (102) ainsi qu'en amont du point de connexion pour l'élément de traitement de sang (103).

5. Dispositif d'administration de sang (100) selon l'une quelconque des revendications 1 à 4,
dans lequel le conduit de sang principal (101) comporte :
- un orifice d'entrée (124) pour un liquide de dilution en amont du point de connexion pour le dialyseur (102) ;
et/ou
- un orifice d'entrée (125) pour un liquide de dilution en aval du point de connexion pour le dialyseur (102) ainsi qu'en amont du point de connexion pour l'élément de traitement de sang (103) ;
et/ou
- un orifice d'entrée (126) pour un liquide de dilution en aval du point de connexion pour l'élément de traitement de sang (103).

6. Dispositif d'administration de sang (100) selon l'une quelconque des revendications 1 à 5,
dans lequel l'élément de traitement de sang (103) est un échangeur de gaz.

7. Système de traitement de sang (1000) pour la mise en œuvre d'un traitement de sang extracorporel comportant un dispositif de traitement de sang (10) et un dispositif d'administration de sang (100) selon l'une quelconque des revendications 1 à 6, dans lequel le dispositif de traitement de sang (10) comporte :
- un dispositif de commande (30) ;
et
- un ensemble de pompe (7), lequel est conçu pour la production de flux sanguins dans le conduit de sang principal (101), ainsi que dans le au moins un conduit secondaire (106), dans lequel l'ensemble de pompe comprend au moins deux éléments, qui agissent sur le flux dans le conduit de sang principal (101) et dans le au moins un conduit secondaire (106) du dispositif d'administration de sang (100),
dans lequel le dispositif de commande (30) est configuré pour faire fonctionner l'ensemble de pompe (7), de telle sorte qu'un premier débit sanguin dans le dialyseur (102) est découplé d'un deuxième débit sanguin dans l'élément de traitement de sang (103), dans lequel en plus
- l'ensemble de pompe (7) est conçu en outre pour la production d'un flux sanguin dans le deuxième conduit secondaire (120),
et
- dans lequel le dispositif de commande (30) est configuré pour faire fonctionner l'ensemble de pompe (7), de telle sorte que le débit sanguin dans au moins une partie du conduit de sang principal (101) est indépendant au moins d'un des débits sanguins dans les conduits secondaires (106, 120).

8. Système de traitement de sang (1000) selon la revendication 7, dans lequel l'ensemble de pompe (7) du dispositif de traitement de sang (10) est réalisé pour produire dans le conduit de sang principal (101) et dans le au moins un conduit secondaire (106) du dispositif d'administration de sang des débits sanguins indépendants les uns des autres.

9. Système de traitement de sang (1000) selon l'une quelconque des revendications 7 ou 8,
dans lequel le dispositif de traitement de sang (10) comporte :
- une pompe à perfusion (110) pour l'amenée d'un liquide médical dans le conduit de sang principal (101),
ou
- deux pompes à perfusion (110, 113) pour l'amenée d'un liquide médical dans le conduit de sang principal (101),
ou
- trois pompes à perfusion (110, 113, 116) pour l'amenée d'un liquide médical dans le conduit de sang principal (101),
ou
- quatre pompes à perfusion (110, 113, 116, 123) ou plus pour l'amenée d'un liquide médical dans le conduit de sang principal (101).

10. Système de traitement de sang (1000) selon la revendication 9,
dans lequel le dispositif de commande (30) du dispositif de traitement de sang (10) est configuré pour réguler la vitesse d'amenée d'au moins une des pompes à perfusion en fonction du débit sanguin dans le dialyseur (102), en particulier dans lequel le dispositif de commande (30) est configuré pour réguler les vitesses d'amenée d'au moins deux des pompes à perfusion respectivement en fonction du débit sanguin dans le dialyseur (102) .

11. Système de traitement de sang (1000) selon l'une quelconque des revendications 7 à 10,
dans lequel le dispositif de traitement de sang (10) comporte un capteur de pression (17) pour la détermination de la pression dans le conduit de sang principal (101) en aval du dialyseur (102) ainsi qu'en amont de l'élément de traitement de sang (103).
